Europäisches Patentamt

European Patent Office

Office européen des brevets

(11) Publication number: 0 178 568
A2

# (12) EUROPEAN PATENT APPLICATION

(21) Application number: 85112704.3

(22) Date of filing: 08.10.85

(51) Int. Cl.⁴: A 61 B 5/14
A 61 B 10/00

(30) Priority: 19.10.84 US 663100

(43) Date of publication of application:
23.04.86 Bulletin 86/17

(84) Designated Contracting States:
BE CH DE FR GB IT LI NL SE

(71) Applicant: ABBOTT LABORATORIES
14th Street and Sheridan Road North St
North Chicago Illinois 60064(US)

(72) Inventor: Houseman, Kenneth R.
309 Ahwahnee Lane
Lake Forest Illinois 60048(US)

(72) Inventor: Parsons, Robert G.
419 Thornapple Lane
Libertyville, IL 60048(US)

(72) Inventor: Ver Lee, Donald J.
563 Drake
Libertyville, IL 60048(US)

(72) Inventor: Vcelka, John L.
905 Wilson Court
Zion, IL 60099(US)

(74) Representative: Modiano, Guido et al,
MODIANO, JOSIF, PISANTY & STAUB Modiano &
Associati Via Meravigli, 16
I-20123 Milan(IT)

(54) Assay device.

(57) A device especially suitable for use in carrying out assays for analytes of interest in various fluids, including a housing with an inlet at one end and a plunger opening at the other, a substantially spherically bead positioned in the housing and adapted to seat in the lower portion of the housing, and a plunger positioned within the housing, the plunger including a head portion and adapted to conform to or deform around and to press the bead into contact with the inlet end of the housing, thereby to ensure that the housing has essentially a zero volume when the pluger is displaced all the way into the housing.

./...

Croydon Printing Company Ltd.

EP 0 178 568 A2

FIG.1

0178568

- 1 -

## Background of the Invention

### Technical Field

This invention relates generally to syringe-like devices, and more particularly to such a device which can advantageously be used in performing assays of biological and other fluids for analytes of interest which may be contained therein.

### Background Art

A wide variety of assays are currently in general clinical use to determine the presence of various analytes, such as antigens or antibodies, in biological samples including human blood, plasma, spinal fluid, urine and the like. Such assays include, for example, enzyme assays and immunoassays, radioimmunoassays and fluorescence polarization immunoassays.

One of the most typical examples of such assays is the enzyme immunoassay conventionally used for determining human hepatitis B surface antigen. In that assay, a serum sample is contacted with a surface, frequently a polystyrene bead coated with a protein or other receptor substance capable of binding or immobilizing the antigen; any antigen present in the sample becomes chemically bound or immobilized upon the coating of the bead. Then, the coated bead is washed to remove serum, leaving the antigen upon the bead surface.

Thereafter, an enzyme labeled antibody to the antigen is contacted with the bead so that the enzyme labeled antibody becomes chemically bound to the antigen on the surface of the bead. Once again, the bead is washed and a final reagent is introduced -- one whicn is capable of developing color, or another detectable response, in the presence of the enzyme which is hence indicative of the presence of the antigen. In the case of a reagent which is a chromogen, development of color can be read by a spectrophotometer, and the absorbance reading thus obtained becomes a measure of the antigen present in the serum sample.

Up to the present time, immunoassay procedures of the type previously described usually have been carried out by laboratory technicians by hand. Not only are such conventional procedures highly labor-intensive, it is not uncommon for samples to be interchanged, thereby resulting in erroneous test results which can have disastrous consequences in patient diagnosis and therapy.

Complete automation of diagnostic immunoassays has heretofore not been successfully accomplished. Partial automation has been accomplished by various systems in which individual reaction containers are part of multiple reaction trays. However, these systems do not provide fully automated testing in that the operator must perform several manual steps: pipeting of samples into individual reaction containers, transfer of multiple reaction trays to incubators, and transfer of multiple reaction trays to instruments that automatically perform various functions.

The primary disadvantages of the prior, partially automated systems are thus the necessity for multiple devices for carrying out the necessary reaction, as well as the necessity for manual intervention in the assay process.

It is, accordingly, an object of the present invention to provide a single device, suitable for carrying out a complete assay procedure.

It is a more specific object of the present invention to provide a diagnostic device which can be employed in conducting assay procedures, particularly immunoassay procedures, in an automated as well as manual fashion, to minimize the efforts and potential errors of operators in carrying out such assays.

It is yet another object of the present invention to provide a diagnostic device which is capable of use in carrying out assay procedures and which ensures highly accurate and reproducible measurements of the presence of analytes, especially antigens or antibodies, in various samples.

These and other objects and advantages will appear more fully hereinafter from the description and accompanying drawings.

## Brief Description of the Drawings

Fig. 1 is a side elevational view in partial cross section of a device of the present invention with the plunger thereof in tne withdrawn position;

Fig. 2 is a view similar to Fig. 1 of the device of the invention wnerein the plunger is partially depressed;

Fig. 3 is an enlarged view in partial cross section taken along the line 3-3 of Fig. 2;

Fig. 4 is a sectional view taken along the line 4-4 of Fig. 3; and

Fig. 5 is an enlarged view in partial cross section of tne device of Fig. 1, showing the plunger fully depressed into the housing.

Fig. 6 is a side elevational view in partial cross section, similar to Fig. 1, of another device according to tne invention.

## Summary of the Invention

The present invention provides a device which can be advantageously used either manually or in an automated fashion to carry out assay procedures, and

particularly immunoassays, wherein great accuracy in the introduction and reaction of various reagents is necessary. The device of the invention includes a body with a housing having inlet means at one end and a plunger opening at the other end. The inlet means includes a bottom portion which substantially closes the housing and which has a substantially hemispherical surface defining the end wall or bottom of the housing. The hemispherical surface includes an opening extending therethrough to permit the passage of fluid into the housing. Also positioned within the housing is a substantially spherical bead which is adapted to be coated with a substance, such as a receptor protein, capable of binding or immobilizing antigen or antibody. The spherical bead is dimensioned to substantially correspond to the hemispherical surface, such that, when the bead is seated in substantial contact with the hemispherical surface, there is substantially zero volume between the bead and the hemispherical surface.

The device of the invention also includes plunger means positioned within the housing and extending outwardly through the plunger opening for access, when the device is in use, to the operator for manual manipulation, or to associated mechanical means capable of automatically manipulating the device. The plunger includes a head portion which is in wiping engagement with the walls of the housing to ensure a fluid-tight seal therebetween. When the plunger is displaced further into the housing, the head portion comes into pressing contact with the bead, and in turn the bead comes into substantial contact with the aforesaid hemispherical surface. The head portion is shaped to either fit snuggly around, substantially conforming to the shape of the bead, or to be deformed about the bead when in substantial contact therewith to provide substantially a zero volume in the housing when the plunger is fully depressed. Thus, in use of the device of the invention to perform an assay, the biological and/or chemical reactions and changes involved in the assay procedures can be carried out efficiently and accurately within the device, either in an automated fashion or manually, resulting in less time and expense, as well as greater assay sensitivity and specificity, by comparison with conventional devices and procedures, with less possibility of operator error.

## Detailed Description of the Invention

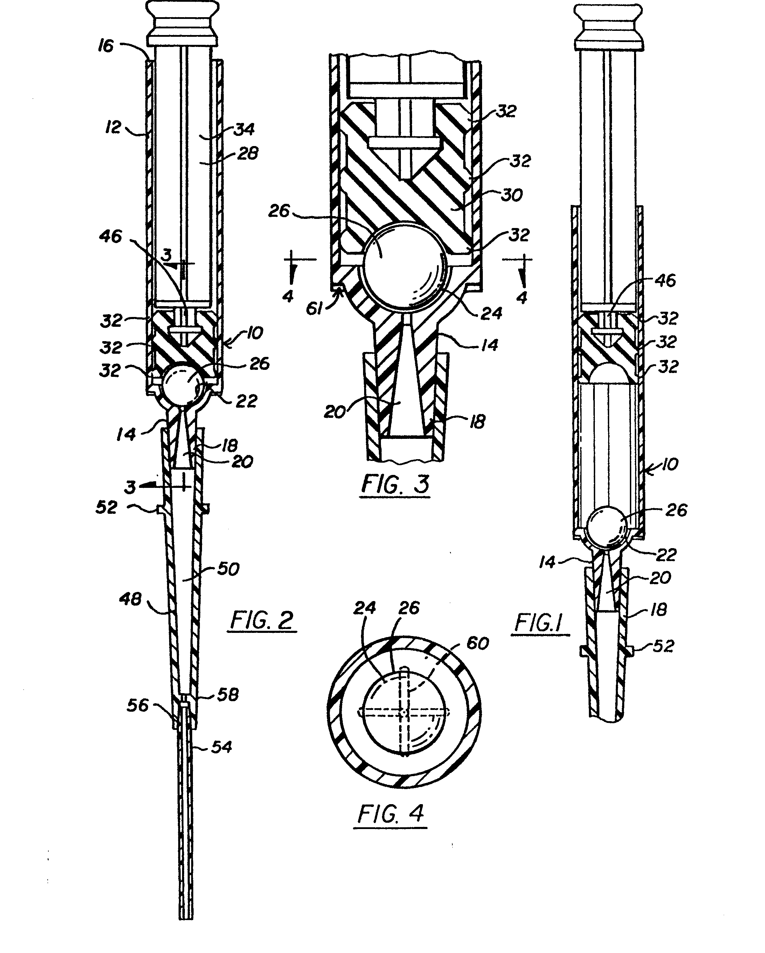

Referring now in more detail to the drawings, Figs. 1 through 5 show a device embodying the concepts of the invention, including a housing 10 having generally cylindrical, longitudinally extending side walls 12, and inlet means 14 at one end of the housing and a plunger opening 16 at the other end of the housing. The inlet means 14 includes a nipple portion 18 integral with the side walls 12 with an opening 20 therethrough, through which a fluid may pass into the housing 10 when the device is in operation. Defining the bottom or end wall of the housing 10 is a wall portion 22 having a substantially hemispherical surface 24.

Positioned within the housing 10 is a substantially spherical bead 26 which is dimensioned essentially to correspond to the shape of the surface 24, so that when the bead 26 is seated in substantial contact with the surface 24, there is substantially zero volume existing between the bead 26 and the surface 24.

Also positioned within the housing 10 is a plunger, generally illustrated at 28, which includes a head portion 30 formed of a resilient elastomeric material, for example a rubber such as isoprene. The head portion 30 includes a plurality of ribs 32 which are in a wiper engagement with the interior surface of the walls 12 of the housing to ensure a fluid-tight seal therebetween.

The plunger 28 also includes an elongated shaft 34 having an actuator handle or knob 36 on the end thereof which can be gripped, either manually or in an automated fashion, to actuate the plunger 28 and move it longitudinally within the housing 10. In this embodiment, the elongated shaft 34 of the plunger 28 is of a different material than the head portion 30, being composed, for example, of a relatively inert polymeric material such as polypropylene or polyethylene. The plunger 28 also includes a mounting element 38 including a central shaft 40 and a mounting head 42. The mounting head 42 includes a pair of flanges 44 and 46 disposed to each other at right angles thereby to secure the deformable head portion 30 to the elongated shaft 34 of the plunger 28.

In the embodiment of the invention shown in Figs. 1-5, the device includes a pipette section 48 in the form of an alongated tube having a tapered central base 50 adapted to frictionally and releasably engage the nipple portion 18 of the inlet means 14. For ease in assembly and disassembly, the pipette section 48 includes a gripping flange 52 having a serrated edge on at least a portion of its periphery to permit the pipette section 48 to be gripped and twisted to release its frictional engagement with the nipple portion 18. The pipette section 48 also includes an inlet tube 54 which is frictionally engaged by a generally tapered opening 56 at the downstream end 58 of the pipette section 48. The inlet tube 54 thus permits, when the device shown is in use, a liquid sample (not shown) to be drawn into the device without contamination of the remainder of the device. The housing 10 also has a serrated edge 61 on at least a portion of its periphery, opposite the serrated edge of the gripping flange 52, for facilitating ease of release of the frictional engagement of the pipette section 48 with the nipple portion 18.

As an example of the use of the device of the invention shown in Figs. 1-5 to perform an immunoassay on a serum sample, the bead 26 positioned within the housing can be, for example, coated with a coating of a receptor for a particular antigen suspected of being in a sample being assayed. Such antigen receptors are themselves well known to those skilled in the art, and form no part of the present invention, as are well-known methodologies for coating such receptors onto such a bead.

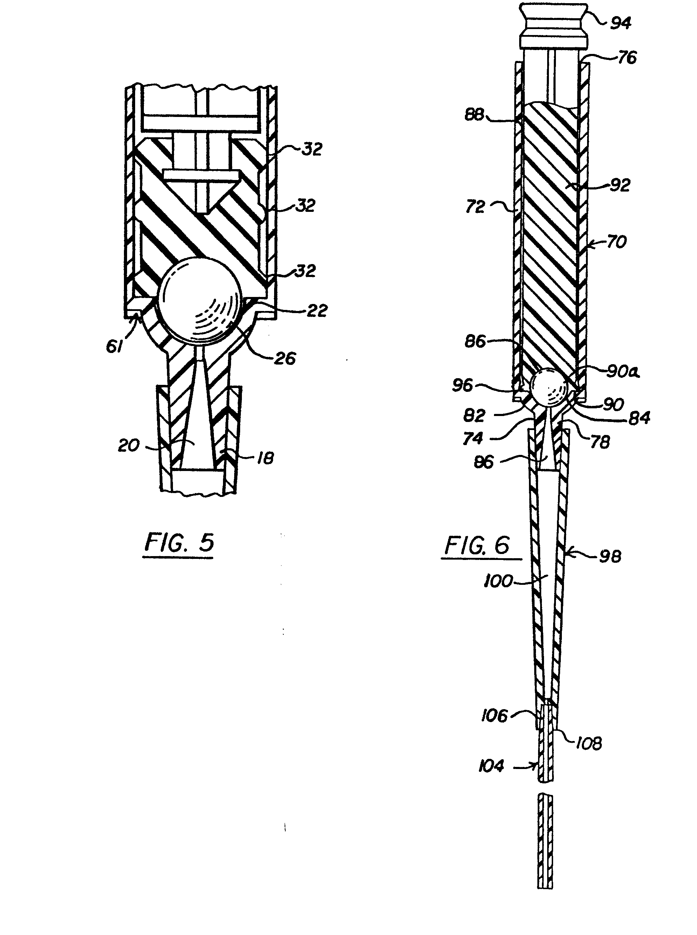

Initially, the plunger 28 is depressed fully into the housing 10 (Fig. 5). Because the head portion 30 of the plunger is deformable, the portion 30 deforms around and assumes substantially the same contour as the bead 26. It is important to this embodiment of the present invention that the head portion 30 so deform because it is important to the success of the assay that the volume in the housing 10 adjacent the bead 26 be essentially zero when the plunger 28 is fully depressed; this situation can only occur, in this embodiment, if the head portion 30 is capable of such deformation. Then, the inlet tube 54 is positioned in a serum sample (not shown) containing the antigen being assayed, and the plunger 28 is withdrawn from the housing, by means of the actuating knob 36, to draw into the pipette section 48 through inlet tube 54 a measured amount of the serum sample. As will be appreciated by those skilled in the art, if the volume of the housing 10 were not substantially zero when the plunger 28 is in the fully depressed position, there is a danger that withdrawal of the plunger 28 to draw in sample fluid could draw in varying amounts of sample, depending on how much free volume existed within the housing 10 before actuation of the plunger 28.

After the sample has been drawn into the pipette section 48 of the device of the invention shown in Figs. 1-5, the inlet tube 54 can be removed, and indeed the bottom portion of the pipette section 48 can be sealed, if desired. It is important, however, that the sample in the pipette section 48 be retained therein and not contact the bead 26 until the desired time, because the reactions which take place between antigen in the sample and the coating on the bead 26 are time-dependent chemical reactions.

At the appropriate time, when it is desired to begin the reactions involved in the assay itself, the sealed pipette section 48 is opened, if necessary, and then the plunger 28 is partially withdrawn from the housing 10 by means of the knob 36, thereby to draw the sample from the pipette section 48 into the housing 10 for contact with the bead 26. Such contact usually occurs under controlled temperature (incubation) and for a measured period of time. The pipette section 48 can then be removed and discarded, if desired. After the reactions are allowed to proceed for the measured period of time, the plunger actuator knob 36 is depressed to fully depress the plunger 28 and to expel from the housing 10 the serum sample, leaving antigen from the sample chemically bound or immobilized on the coating on the bead 26. The action of the plunger 28, which deforms the head portion 30 thereof around the bead 26 in expelling a liquid through passage 20, again returns the fluid volume of the housing 10 around the bead 26 to essentially zero to ensure that all of the serum sample has been expelled from the housing. In that regard, it is preferred, in accordance with the practice of the invention, that the substantially hemispherical surface 24 include a plurality of grooves therein 60 (Fig. 4) to provide passage means between the surface 24 and the bead 26 through which any liquid remaining can be drained and expelled from the housing 10.

After the serum has been expelled, the bead 26 is washed by providing to the nipple portion 14 a source of wash water (not shown) which is drawn into the housing 10 through the passage 20 to wash any remaining serum from the bead 26, again leaving the antigen bound or immobilized upon the coating on the bead 26. To ensure adequate mixing of the wash water and any remaining reagents in the housing 10, the passage 20 preferably has a conical or tapered configuration to create turbulence in the fluid being drawn through the housing 10 as the plunger 28 is withdrawn. Thus, the plunger 28 is withdrawn from a portion of the housing 10 to draw the wash water into the housing 12 to wash the bead 26, as described above.

After washing of the bead 26, the wash water is expelled from the housing 10 by again fully depressing the plunger 28 to the full extent to again create essentially zero volume in the housing 10 as the head portion 30 of the plunger 28 is deformed about the bead 26.

After the wash water has been expelled, the plunger 28 is again actuated by means of actuating knob 36 to draw into the housing 10 a measured amount of an enzyme labeled antibody solution, which is contacted in the housing 10 with the bead 26 to chemically react with the antigen bound to the coating on the bead 26. After the enzyme labeled antibody solution is allowed to contact for a measured time the surface coating on the bead 26, it is expelled by again fully depressing the plunger 28 to reduce the volume in the housing 10 to essentially zero, and the bead 26 is again washed in the same manner as described above.

Finally, the last reagent, a chromophore reagent, is drawn into the housing 10, by again actuating the plunger 28 as aforedescribed, for reaction to the presence of the antigen-enzyme labeled antibody coating on the bead 26. The chromophore develops color in response to the presence of the enzyme, as a measure of the antigen concentration in the serum. The color thus developed in the solution contained in the housing 10 can be measured by means well known to those skilled in the art, such as spectrophotometrically, to ascertain the concentration of antigen present in the original sample.

Referring now to Fig. 6 of the drawings, a preferred embodiment of the device of the invention is shown. This preferred device comprises a housing 70 having generally cylindrical, longitudinally-extending side walls 72, and inlet means 74 at one end of the housing 70 and a plunger opening 76 at the other end. The inlet means 74 includes a nipple portion 78 integral with the side walls 72 with an opening 80 therethrough, through which a fluid may pass when the device is in operation. Defining the bottom or end wall of the housing 70 is a wall portion 82 having a substantially hemispherical surface 84.

Positioned within the housing 70 is a substantially spherical bead 86 which is dimensioned to correspond to the shape of the surface 84, so that when the bead 86 is seated in substantial contact with the surface 84, there is essentially zero volume existing between the bead 86 and the surface 84.

Also positioned within the housing 70 is a plunger, generally illustrated at 88, which includes a head end 90 and an elongated shaft 92 having an actuator handle or knob 94 on the end thereof which can be gripped, either manually or in an automated fashion, to actuate the plunger 88 and move it longitudinally within the housing 70. The head end 90 includes ribs 96 which are in wiper engagement with the interior surface of the walls 72 of the housing 70 to ensure a fluid-tight seal therebetween.

In this preferred embodiment, tne plunger 88 is formed of a relatively inert polymeric material such as polypropylene, and is of one-piece construction, tne head end 90 having a nemispherically shaped surface 90a substantially corresponding to tne shape of the bead 86.

Therefore, in use of this preferred device to perform an assay, the steps of wnich assay can be substantially as previously described with respect to the device shown in Figs. 1-5, when the plunger 88 is fully depressed, essentially zero volume is achieved within the housing 70 because the hemispherical surface 90a substantially contacts the bead 86 and, suostantially conforming to the shape thereof, provides essentially no space within the housing 70 in which fluid can remain.

In the preferred embodiment of the invention snown in Fig. 6, the device also includes a pipette section 98 in the form of an elongated tube having a tapered central bore 100 adapted to frictionally engage the nipple portion 78 of the inlet means 74. For ease in assembly and disassembly, the pipette section 98 further includes a gripping flange 102 having a serrated edge on at least a portion of its periphery to permit the pipette section 98 to be gripped and twisted to release its frictional engagement with the nipple portion 78. The pipette section 98 also includes an inlet tube 104 which is frictionally engaged by a generally tapered opening 106 at the downstream end 108 of the pipette section 98. 'As previously described with respect to the device of Figs. 1-5, the inlet tube 104 functions, in use, to permit liquid (not shown) to be drawn into the device.

As previously mentioned, operation of the preferred device of the invention shown in Fig. 6 to perform, for example, an immunoassay on a serum sample, is accomplished in substantially the same manner as previously described for use of the device shown and described with reference to Figs. 1-5. However, it is to be appreciated that this preferred device differs from the device of Figs. 1-5 in having a one-piece plunger instruction, wherein the head end 90 of the plunger 88 is not made of an elastomeric material which is deformable around the bead 86, but rather conforms to the shape thereof because of the shape of the surface 90a. Thus, in this preferred embodiment the head end 90 of the plunger 88 is of the same material as the shaft 92 only is advantageously molded, during its fabrication, to be integral therewith. Accordingly, while an assay can be performed in this preferred device substantially as previously described, this preferred device possesses a simpler structure which, because of its internal configuration, in use enables the advantages aforementioned, while permitting the device to be advantageously assembled with fewer components.

It is to be appreciated that the selection of particular materials for the components comprising a device of the invention will be largely a matter of choice for the routineer, given the disclosure hereof. However, if highly sensitive immunoassays such as aforedescribed are to be carried out in the device, one skilled in the art will readily recognize that materials should be selected which are substantially non-reactive to the biological samples to be analyzed and to the various reagents which must be employed in such analyses. For example, it is preferred in fabricating devices intended for immunoassays that known relatively inert polymeric materials be employed, such as polypropylene, polyethylene, polystyrene, polyacrylamides and the like. In addition, where the intended use will permit, one can use various glass and glass-like materials, resinous materials, fiberglass, and rubbers such as isoprene, butylene and other elastomeric materials for the whole or a part of the device. Many other suitable materials will become apparent to the routineer, given the teachings hereof and the use contemplated for a particular device made in accordance therewith.

It is apparent that not only can immunoassays, in particular enzyme immunoassays for an antigen or antibody of interest, be advantageously carried out utilizing devices of the invention, but that in addition many other chemical and biological assays, reactions and procedures can be accomplished. For example, devices of the invention may be employed in such assays as dual sequential enzyme assays for various analytes, in direct assays for various ions in fluids such as water, blood or urine, or for many other uses. All such uses are within the scope of application of the present invention, and it is to be appreciated that this invention, especially in view of the aforedescribed advantages thereof can have a myriad of applications.

- 14 -

As is apparent from the foregoing description, the device of the present invention enables the entire series of chemical reactions and changes which are necessary in the performance of an assay to take place in a single reaction chamber, namely, for example, within the housing 10 of the device of the invention illustrated in Figs. 1-5, or within the housing 70 of the preferred device shown in Fig. 6. Accordingly, the device can be used either manually, e.g., by a laboratory technician, or can be employed in automated equipment capable of processing large quantities of such devices to perform assays in a continuous manner. Such automated equipment, while not part of the present disclosure, can be of a type designed to effect programmed manipulation of the plunger of the device in response to a sequence of steps in an assay procedure which have been set by electronic control means associated therewith.

It is to be appreciated that although devices of the invention have been described which contain a substantially spherical bead, upon which a substance is bound or immobilized, which is not an integral part of the plunger of the device, it is contemplated that a device in accordance with the invention may take the form of one wherein such a bead is attached to or fabricated as an integral part of the plunger. Thus, it will be understood that this and various other changes and modifications can be made in the details, procedure, construction and use of the devices specifically described herein without departing from the spirit and scope of the invention, which shall be defined solely by the following claims.

CLAIMS:

1.   A device comprising

(a) a body including a housing having inlet means at one end thereof and a plunger opening at the other end tnereof,

(b) the inlet means including a substantially hemispherical surface defining an end wall of the housing and an opening extending therethrough adapted to permit tne passage of fluid into the housing,

(c) a suostantially spherical bead positioned in the housing and dimensioned to suostantially correspond to the hemispherical surface, so that when said bead is seated in suostantial contact with tne hemispherical surface, there is essentially zero volume between said bead and hemispherical surface.

2.   A device as defined in Claim 1, wherein the inlet means includes a nipple portion having a central passage therein communicating with the housing and being integral therewith.

3.   A device as defined in Claim 2, wherein the central passage nas a conical configuration, thereby to create turbulence in fluids passing therethrougn into the housing.

4.   A device as defined in Claim 1, further including a pipette section releasaoly mounted on said body, said pipette section having a central opening communicating with the inlet meàns.

5.   A device as defined in Claim 4, wherein said pipette section includes gripping flange means for removing said pipette section from the inlet means of the housing.

6.   A device as defined in Claim 1, wherein said bead contains a coating of an antigen receptor on the surface thereof.

7.   A method for performing an assay for an analyte in a fluid, comprising carrying out the assay in the device claimed in any one of Claims 1-6.

FIG. 1

FIG. 2

FIG. 3

FIG. 4

2/2

FIG. 5

FIG. 6